# FASCICULE DE BREVET EUROPEEN

(11) **EP 1 022 265 B1**
(45) Date de publication et mention de la délivrance du brevet: **26.03.2003**
(21) Numéro de dépôt: 99125371.7
(22) Date de dépôt: 20.12.1999
(51) Int. Cl.: C07C 33/34, C07C 29/145, C07C 49/252, C07C 45/75, C11B 9/00, A61K 7/46

(54) **Dérivés du 2-indaneméthanol et leur utilisation à titre d'ingrédients parfumants**
2-Indanmethanolderivate und ihre Verwendung als Riechstoffe
2-Indanemethanol derivatives and their use as perfuming ingredients

(30) Priorité: 22.01.1999 CH 12399
(43) Date de publication de la demande: 26.07.2000
(73) Titulaire: FIRMENICH SA, 1211 Genève 8 (CH)
(72) Inventeur: Winter, Beat, 1233 Bernex (CH); Schneider, Philippe, 1208 Geneve (CH)
(74) Mandataire: Salvaterra-Garcia, Maria de Lurdes, Dr.

(56) Documents cités:
- EP-A- 0 365 015
- WO-A-98/55439
- GB-A- 2 233 645
- SONNENBICHLER J ET AL: "Secondary fungal metabolites and their biological activities. IV. Synthesis of compounds with structural similarities to the toxic metabolites of the pathogenic fungus Heterobasidion annosum and investigation of their antibiotic activities" BIOL. CHEM. HOPPE-SEYLER (BCHSEI,01773593);1993; VOL.374 (11); PP.1047-55, XP000874559 Max-Planck-Inst. Biochem.;Martinsried; Germany (DE)

## Description

La présente invention a trait au domaine de la parfumerie. Elle concerne plus particulièrement l'utilisation d'alcools de formule générale 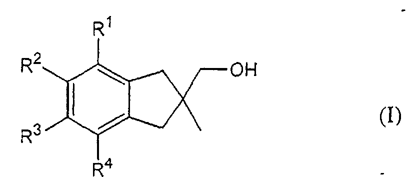 dans laquelle les symboles R¹, R², R³ et R⁴ représentent, indépendamment l'un de l'autre, un atome d'hydrogène ou un radical alkyle inférieur de C₁ à C₃, linéaire ou ramifié, saturé ou insaturé, à titre d'ingrédients parfumants.

Les composés de l'invention présentent des caractéristiques odorantes de type muguet, très utiles pour la parfumerie. En outre, ils présentent de nombreux avantages tels qu'une bonne stabilité dans certains milieux agressifs pour le parfum, ainsi qu'une excellente ténacité, ce qui les rend particulièrement utiles et appréciables pour des applications dans le domaine de la parfumerie fonctionnelle. Ils peuvent de ce fait, servir à préparer des parfums, compositions parfumantes et articles parfumés.

Les alcools de formule (I) développent une odeur de type muguet, frais, humique très agréable. Cette odeur est proche de celle de l'hydroxycitronellal (7-hydroxy-3,7-diméthyloctanal), bien que plus puissante que cette dernière. Son caractère peut être également décrit comme se situant entre celui du Liliale [3-(4-tert-butylphényl)-2-méthylpropanal] et celui du Lyral® [4-(4-hydroxy-4-méthylpentyl)-3-cyclohéxène-1-carbaldéhyde].

Le composé de formule (I) dans laquelle R¹, R², R³ et R⁴ représentent chacun un atome d'hydrogène, à savoir le 2-méthyl-2-indaneméthanol possède une structure connue en soi. Sa synthèse est décrite dans la littérature par J. Sonnenbichler et al., Biol. Chem. Hoppe-Seyler (1993), 374(11), 1047-55, par Shono et al., Journal of Org. Chem. (1982), 47(16), 3090-4 ou encore par Liu et al., Tetrahedron Lett. (1979), (43), 4121-4. On ne trouve cependant dans ces références, ni une description de l'odeur du 2-méthyl-2-indaneméthanol, ni une suggestion quant à sa possible utilisation en parfumerie.

Par ailleurs, la demande de brevet européen EP 685 444 décrit des aldéhydes dont certains ont une structure bicyclique apparentée à celle des composés de l'invention. Sont cités notamment, le 5-tert-butyl-2-indanecarbaldéhyde ou encore le 6-tert-butyl-1-indaneacétaldehyde. Cette demande fait état des propriétés odorantes de ces composés et de leur utilisation dans le domaine de la parfumerie. Bien que ces composés connus soient plus stables que d'autres aldéhydes antérieurs, les alcools de formule (I) selon la présente invention se révèlent encore plus stables et avantageux tout en ayant des odeurs tout aussi utiles, qui sont par ailleurs d'une ténacité très avantageuse. Leurs propriétés odorantes diffèrent de surcroît de celles de ces aldéhydes connus et ils apportent des nuances odorantes nouvelles à la palette du parfumeur.

La demande de brevet européen EP 365 015 décrit le 1,1-diméthyl-3-hydroxyméthylindane ainsi que son utilisation dans le domaine de la parfumerie. Les propriétés odorantes diffèrent des propriétés odorantes des composés selon formule (I).

Les composés de formule (I), mis à part le 2-méthyl-2-indaneméthanol, sont par ailleurs nouveaux et l'invention concerne également ces composés.

Parmi les composés de l'invention, les composés de formule (I) dans laquelle seuls deux symboles choisis parmi R¹, R², R³ et R⁴ représentent un atome d'hydrogène, les 2 autres étant différents, sont plus appréciés. On peut citer en particulier le 2,5,6-triméthyl-2-indaneméthanol ou encore le 2,4,6-triméthyl-2-indaneméthanol qui possèdent tous les deux une odeur fleurie de type muguet, rappelant certaines notes du Lilial® et de l'hydroxycitronellal.

Par ailleurs, on apprécie également les composés (I) de l'invention, caractérisés en ce que seul l'un des symboles R¹ à R⁴ est différent de l'hydrogène. Ainsi, le 2,4-diméthyl-2-indaneméthanol possède une odeur également fleurie de type muguet, de même que le 2,5-diméthyl-2-indaneméthanol, composé préféré selon l'invention.

L'odeur de ce dernier est de type muguet, frais, proche de l'odeur de l'hydroxycitronellal (7-hydroxy-3,7-diméthyloctanal), avec en note de fond un caractère humique, muguet, très apprécié, rappelant l'odeur caractéristique du Mayol® (cis-7-p-menthanol ; origine : Firmenich SA. Genève, Suisse). Ce composé se distingue particulièrement de l'hydroxycitronellal par son excellente ténacité qui lui confère une grande valeur pour son utilisation en parfumerie. Celle-ci s'est révélée être supérieure à celle des aldéhydes connus cités plus haut, mais elle est également nettement supérieure a celle du Mayol® (cis-7-p-menthanol). Cette caractéristique a été confirmée en lavage, sur linge humide et sec.

Les propriétés organoleptiques citées plus haut sont relatives aux composés présents sous forme racémique. Or, en raison de leur structure, les composés (I) peuvent également se présenter sous forme optiquement active et montrer des nuances olfactives distinctes des composés racémiques, permettant ainsi d'obtenir des fragrances variées selon que l'on utilise l'un ou l'autre des isomères. Par exemple, alors que l'odeur du (+)-2,5-diméthyl-2-indaneméthanol dans sa note muguet, fleurie, rappelle plutôt le Lilial® en note de fond, le (-)-2,5-diméthyl-2-indaneméthanol est plus proche de l'hydroxycitronellal.

Les composés de l'invention se prêtent aussi bien à une utilisation en parfumerie fine, dans les parfums, eaux de toilette ou lotions après-rasage, qu'à d'autres emplois courants en parfumerie tels que le parfumage des savons, gels de douche ou de bain, de produits d'hygiène ou de traitement des cheveux comme les shampoings, ainsi que de déodorants corporels et déodorants ambiants, ou encore de préparations cosmétiques.

Les composés (I) peuvent également être employés dans les applications telles que détergents liquides ou solides destinés au traitement des textiles, adoucissants textiles, ou encore compositions détergentes ou produits d'entretien destinés au nettoyage de la vaisselle ou de surfaces variées, domestiques ou industrielles.

Dans ces applications, les composés de l'invention peuvent être utilisés seuls ou en mélange avec d'autres ingrédients parfumants, des solvants ou adjuvants d'usage courant en parfumerie. La nature et la variété de ces coingrédients n'appellent pas une description plus détaillée ici, qui ne saurait d'ailleurs être exhaustive, l'homme du métier étant à même de les choisir de par ses connaissances générales et en fonction de la nature du produit à parfumer et de l'effet olfactif recherché.

Ces ingrédients parfumants appartiennent à des classes chimiques aussi variées que les alcools, aldéhydes, cétones, esters, éthers, acétates, nitriles, hydrocarbures terpéniques, composés hétérocycliques azotés ou soufrés, ainsi que des huiles essentielles d'origine naturelle ou synthétique. Beaucoup de ces ingrédients sont d'ailleurs répertoriés dans des textes de référence tels que le livre de S. Arctander, Perfume and Flavor Chemicals, 1969, Montclair, New Jersey, USA, ou ses versions plus récentes, ou dans d'autres oeuvres de nature similaire.

Les proportions dans lesquelles les composés selon l'invention peuvent être incorporés dans les produits divers susmentionnés varient dans une gamme de valeurs étendue. Ces valeurs dépendent de la nature de l'article ou produit que l'on veut parfumer et de l'effet olfactif recherché, ainsi que de la nature des coingrédients dans une composition donnée lorsque les composés de l'invention sont utilisés en mélange avec des coingrédients parfumants, des solvants ou des adjuvants d'usage courant dans l'art.

A titre d'exemple, on peut citer des concentrations typiques de l'ordre de à 10% en poids, voire même 20% ou plus en poids, des composés (I) de l'invention, par rapport au poids de composition parfumante dans laquelle il est incorporé. Des concentrations bien inférieures à celles-ci peuvent être utilisées lorsque les composés sont directement appliqués dans le parfumage des produits de consommation divers cités auparavant.

L'invention concerne également un procédé général pour la préparation des composés selon l'invention, caractérisé en ce qu'on effectue une hydrogénolyse de l'indanone de formule 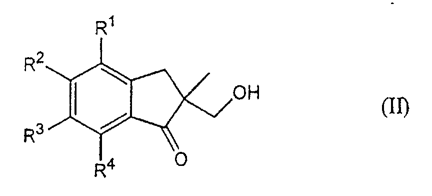 dans laquelle les symboles R¹, R², R³ et R⁴ sont définis comme à la formule (I).

Cette étape est effectuée dans des conditions propres à ce type de réaction, qui sont connues de l'homme du métier. L'indanone de départ est préparée à partir de composés connus ou qui peuvent être obtenus de façon conventionnelle, à l'aide d'une réaction d'aldol avec formaldéhyde (sous une de ses formes quelconques), qui peut être représentée par le schéma suivant :

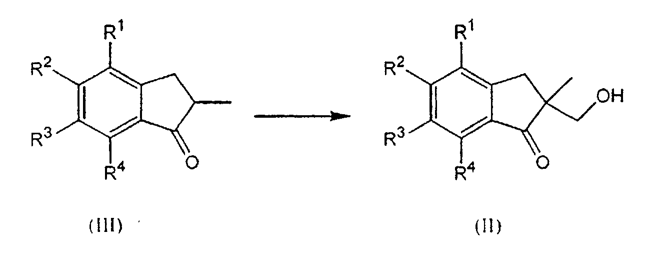

Les composés de formule (II) sont des composés nouveaux et constituent un objet de l'invention.

L'invention sera maintenant décrite de façon plus détaillée dans les exemples suivants dans lesquels les températures sont indiquées en degrés Celsius et les abréviations ont le sens usuel dans l'art.

### Exemple 1

### Préparation du 2,5-diméthyl-2-indaneméthanol

### i) Préparation du mélange racémique

La synthèse du 2,5-diméthyl-2-indaneméthanol a été effectuée en deux étapes à partir de la 2,6-diméthyl-1-indanone. La synthèse de cette dernière a été décrite par littérature (voir H.W. Pinnick, S.P. Brown, E.A. McLean, L.W. Zoller III, dans J. Org. Chem., 1981, 46, 3758).

### a) Préparation de la 2-hydroxyméthyl-2,6-diméthyl-1-indanone à partir de la 2,6-diméthyl-1-indanone

Dans un récipient de type Schmizo de 1.5 1 équipé d'un réfrigérant, d'un agitateur mécanique et sous azote, on a introduit 147,53 g de K₂CO₃, 875 g de toluène et 350 g de 2,6-diméthyl-indanone. Le mélange a été chauffé à 51°. On a ensuite ajouté 122,45 g de Formcel® 55% (origine : Hoechst) en 1 h. Le mélange a été maintenu sous agitation à 51° pendant 2 h. On a alors refroidi le milieu réactionnel à 24° et ajouté 350 g d'eau. Après avoir agité pendant 10 min, on a décanté, puis lavé 3 fois avec de l'eau. Une concentration sous vide a permis d'obtenir 404,71 g de produit brut pur à 97,6% (CG).

### Données analytiques :

IR (CHCl₃) : 3430, 2910, 1685, 1600, 1485, 1380, 1035, 810 cm⁻¹
RMN(¹H, 360 MHz, CDCl₃) : 7,48(s, 1H) ; 7,40(d, J=8, 1H) ; 7,33(d, J=8, 1H) ;3,82(d, J=11, 1H) ; 3,60(d, J=11, 1H) ; 3,22(d, J=16, 1H) ; 2,82(d, J=16, 1H) ; 2,78(large, OH) ; 2,36(s, 3H) ; 1,20(s, 3H)
RMN(¹³C, 90,5 MHz, CDCl₃) : 211,4(s) ; 150,8(s) ; 137,4(s) ; 136,5(d); 136.0(s) ; 126,3(d) ; 124,1(d) ; 67,6(t) ; 51,3(s) ; 37,6(t) ; 21,0(q) ; 20.7(q)
SM : 190(M⁺,56), 175(65), 172(78), 159(100), 145(72), 129(73), 115(62), 104(27). 91(44), 77(33), 63(18), 51(20), 39(19), 31(21)

### b) Préparation du 2,5-diméthyl-2-indaneméthanol à partir de la 2-hydroxyméthyl-2,6-diméthyl-1-indanone

On a ajouté à une solution de produit obtenu sous a) (200 g) dans de l'isopropanol (3000 g), du Pd/C à 5% (20 g), de l'acide méthanesulfonique (2 g) et on a mélangé sous H₂ (3 x10⁵ Pa) à 80° durant 7h30. Le mélange a été repris dans le toluène (200 g) et lavé successivement avec H₂SO₄ à 5%, H₂O, NaHCO₃ et H₂O, puis concentré sous vide et enfin distillé sur résidu (0,08 x 10² Pa) pour obtenir 161,2 g de produit final sous forme d'un liquide visqueux incolore, avec une pureté de 95,4% (CG).

### Données analytiques :

IR(pur) : 3330(large), 2910, 1485, 1035, 810 cm⁻¹
RMN (¹H, 360 MHz, CDCl₃) : 7,04(d, J=8, 1H) ; 6,98(s, 1H) ; 6,93(d, J=8, 1H) ; 3,49(s, 2H) ; 2,87(d. J=16, 1H) ; 2,85(d, J=16, 1H) ; 2,60(d, J=16, 2H) ; 2,30(s, 3H) ; 1,80(s, OH) ; 1,16(s, 3H)
RMN(¹³C, 90,5 MHz, CDCl₃) : 142,6(s) ; 139,4(s) ; 135,8(s) ; 127,0(d) ; 125,5(d) ; 124,5(d) ; 70,5(t) ; 45,0(s) ; 42,7(t) ; 42,4(t) ; 24,0(q) ; 21,2(q)
SM : 176(M⁺, 33), 158(11), 143(100), 128(35), 115(21), 105(11), 91(11), 77(8), 51(6), 39(7), 31(15)

### ii) Séparation des 2 isomères optiquement actifs

Les deux énantiomères du 2,5-diméthyl-2-indaneméthanol ont été séparés analytiquement par HPLC (chromatographie liquide à haute pression) sur une colonne chirale Chiralpack ® AD (Amylose-tris-3,5-diméthylphényle carbamate, Daicel). La séparation a permis d'obtenir les énantiomères sous forme pure, avec un excès énantiomérique supérieur à 97%.

### Données analytiques :

(+)-2,5-diméthyl-2-indaneméthanol
[α]^{D}₂₀ = +3,2° (dans CHCl₃ à 1,5%)
ee = 99,4% (par HPLC)
Pureté CG : > 99,9%
(-)-2,5-diméthyl-2-indaneméthanol
[α]^{D}₂₀ = -3,1° (dans CHCl₃ à 1,5%)
ee = 97,3% (par HPLC)
Pureté CG :> 99,7%

### Exemple 2

### Préparation du 2,4-diméthyl -2 -indaneméthanol

La synthèse du 2,4-diméthyl-2-indaneméthanol a été effectuée en deux étapes à partir de la 2,7-diméthyl-1-indanone. La synthèse de cette dernière a été décrite par M. Fukuoka et al. dans Chem. Pharm. Bull., 1983, vol. 31, p. 3113.

### a) Préparation de la 2-hydroxyméthyl-2,7-diméthyl-1-indanone à partir de la 2,7-diméthyl-1-indanone

On a ajouté 0,51 g (3,7 mmol) de K₂CO₃ à une solution sous agitation de 1,17g (7,3 mmol) de 2,7-diméthyl-1-indanone dans ml de toluène. Le mélange ainsi obtenu a été chauffé à 50°. Goutte à goutte, 0,42 ml (7,7 mmol) de Formcel® à 55% (origine Hoechst) ont été ajoutés, provoquant une légère exothermie, et le mélange a été maintenu sous agitation à 50° durant 4,5 h. Le mélange a ensuite été versé dans un bain de saumure et d'éther ; la phase organique a été lavée 2 fois à l'aide de saumure, puis séchée sur Na₂SO₄ et concentrée pour obtenir 1,42 g de sirop. Une cristallisation à partir d'un mélange éther-pentane à -30° a permis d'obtenir 0,77 g de cristaux incolores (p.f. 93,5-94°) et 0,51 g de liqueur-mère solide. Au total on a donc obtenu 1,29 g de produit avec une pureté supérieure à 99% et un rendement de 92%.

### Données analytiques :

IR(CHCl₃) : 3580, 3470, 2990, 2950, 2910, 2860, 1680, 1585, 1470, 1370, 1325, 1260, 1190, 1035, 980, 920 cm⁻¹
RMN (¹H, 360 MHz, CDCl₃) : 7,44(t, J=6, 1H); 7,27(d, J=6, 1H); 7,10(d, J=6, 1H) ; 3,79(dd, J₁=10, J₂=6, 1H) ; 3,60(dd, J₁=10, J₂=6, 1H) ; 3,18(d, J=16, 1H) ; 2,83(d, J=16, 1H) ; 2,61(s, 3H) ; 2,57(m, OH) ; 1,22 (s, 3H)
RMN(¹³C, 90,5 MHz, CDCl₃) : 212,1(s) ; 153,9(s) ; 139,4(s) ; 134,4(d) ; 133,2(s) ; 129,3(d) ; 124,0(d) ; 67,9(t) ; 50,7(s) ; 37,5(t) ; 20,9(q) ; 18,3(q)
SM: 190(M⁺, 87), 175(33), 172(44), 159(100), 145(54), 129(41), 115(38), 103(11), 91(22), 77(13), 63(6), 51(5), 39(4)

### b) Préparation du 2,4-diméthyl-2-indaneméthanol à partir de la 2-hydroxyméthyl-2,7-diméthyl-1-indanone

On a ajouté, à une solution de 0,67 g (3,5 mmol) de produit obtenu sous *a)* dans 10 ml d'acide acétique, 0,1 g de Pd-C à 10% et le mélange a été agité vigoureusement sous atmosphère H₂ (10⁵ Pa) à température ambiante durant 6 jours. Le catalyseur a été filtré et le filtrat concentré sur rotavapeur. Le résidu obtenu a été évaporé trois fois à partir de toluène pour finalement laisser place à 0,62 g d'une huile opaque contenant 91% de l'alcool désiré et 8% de l'acétate correspondant. Ce produit brut a été absorbé dans 1 ml de mélange de saponification (2,5 N NaOH dans EtOH-H₂O 1 : 1), puis chauffé à reflux (température de bain 108°) durant 1 h. Le mélange refroidi a ensuite été versé dans un mélange de saumure et éther, la phase organique a été lavée à l'aide de NaHCO₃ sat. aq., et de saumure, puis séchée sur Na₂SO₄ et concentrée pour obtenir 0,60 g d'une huile jaune. Une distillation sur four à boules (0,2x10² Pa, température de four 75-135°) a permis d'obtenir 0,51 g de 2,4-diméthyl-2-indaneméthanol avec une pureté supérieure à 99% et un rendement de 83%.

### Données analytiques :

IR(pur) : 3320(large), 3020, 2900, 1585, 1455, 1370, 1030, 760 cm⁻¹
RMN(¹H, 360 MHz, CDCl₃) : 7,04(t, J=7, 1H) ; 6,99(d, J=7, 1H) ; 6,94(d, J=7, 1H) ; 3,50(s, 2H) ; 2,91(d, J=16, 1H) ; 2,83(d, J=16, 1H) ; 2,67(d, J=16, 1H) ; 2,58(d, J=16, 1H) ; 2,21(s, 3H) ; 1,84(large, OH) ; 1,18(s, 3H)
RMN(¹³C, 90,5 MHz, CDCl₃) : 142,2(s) ; 141,3(s) ; 134,1(s) ; 127,0(d) ; 126,4(d) ; 122,1(d) ; 70,8(t) ; 44,2(s) ; 43,0(t) ; 41,5(t) ; 24,3(q) ; 19,0(q)
SM : 176(M⁺, 32), 158(14), 143(100), 128(32), 115(18), 105(12), 91(10), 77(8), 51(6), 39(8), 31(7), 27(5)

### Exemple 3

### Préparation du 2,5,6-triméthyl -2 -indaneméthanol

La synthèse du 2,5,6-triméthyl-2-indaneméthanol a été effectuée en deux étapes à partir de la 2,5,6-triméthyl-1-indanone. La synthèse de cette dernière a été décrite par M. Fukuoka et al. dans Chem. Pharm. Bull., 1983, vol. 31. p. 3113.

### a) Préparation de la 2-hydroxyméthyl-2,5,6-triméthyl-1-indanone à partir de la 2,5,6-triméthyl-1-indanone

On a ajouté à température ambiante 0,97 g (10,7 mmol) de 1,3,5-trioxane (origine : Fluka) à une solution sous agitation de 4,22g (21,6 mmol) de 2,5,6-triméthyl-1-indanone dans 20 ml d'acide trifluoroacétique. Le mélange ainsi obtenu a été chauffé à 50° pendant 19 h. Une fois refroidi, le mélange a été dilué avec de l'éther puis lavé avec de l'eau et de la saumure, la phase organique a été séchée sur Na₂SO₄ et concentrée pour obtenir un liquide brun. Une distillation sur four à boules (0,35x10² Pa, température du four 125-145°) a permis d'obtenir 5,4 g de trifluoroacétate correspondant. Ce produit a été dissous dans 12 ml d'une solution 2,5 N de NaOH dans EtOH-H₂O 1 : 1, puis chauffé à reflux durant 1 h. Une fois refroidi, le mélange a été dilué avec de l'éther et de l'eau, la phase organique a été lavée avec un solution aqueuse saturée de NaHCO₃ et de la saumure, puis séchée sur Na₂SO₄ et concentrée pour donner 4,2 g d'une huile jaune contenant 30% d'indanone et 59% de céto-alcool. Une distillation sur four à boules (0,3x10² Pa, température du four 75-145°) a fourni 1,95 g d'une fraction enrichie en 2-hydroxy-2,5,6-triméthyl-1-indanone avec rendement de 32%.

### Données analytiques :

IR(pur) : 3420, 2960, 2920, 2860, 1680, 1600, 1450, 1410, 1340, 1360, 1080, 950, 920, 890, 820 cm⁻¹
RMN(¹H, 360 MHz, CDCl₃) : 7,47(s, 1H) ; 7,22(s, 1H) ; 3,80(d, J=10, 1H) ; 3,59(d, J=10, 1H) ; 3,16(d, J=16, 1H) ; 2,80(d, J=16, 1H) ; 2,65(large s, OH) ; 2,33(s, 3H) ; 2,27(s, 3H) ; 1,21(s, 3H)
RMN(¹³C, 90,5 MHz, CDCl₃) : 211,1(s) ; 151,6(s) ; 145,6(s) ; 136,4(s) ; 133,9(s) ; 127,4(d) ; 124,5(d) ; 67,7(t) ; 51,0(s) ; 37,6(t) ; 20,8(q) ; 20,7(q) ; 19,7(q)
SM : 204(M⁺, 66), 189(74), 186(52), 173(100), 159(83), 143(42), 128(48), 115(50), 105(14), 91(31), 77(21), 63(11), 51(13), 39(14), 31(15)

### b) Préparation du 2,5,6-triméthyl-2-indaneméthanol à partir de la 2-hydroxyméthyl-2,5,6-triméthyl-1-indanone

On a ajouté, à une solution de 1,79 g (6,8 mmol) de produit obtenu sous *a)* dans 35 ml d'acide acétique, 0,17 g de Pd-C à 10% et le mélange a été agité vigoureusement sous atmosphère H₂ (10⁵ Pa) à température ambiante durant 12 jours. Le mélange a été filtré sur célite et concentré sur rotavapeur. Le résidu obtenu a été évaporé trois fois à l'aide de toluène pour finalement donner 1,9 g d'une huile contenant 60% de l'alcool désiré, 11% de l'acétate correspondant et 11% de diol intermédiaire. Ce produit brut a été dilué dans 2 ml d'une solution 2,5 N NaOH dans EtOH-H₂O 1 : 1, puis chauffé à reflux durant 1 h. Le mélange a ensuite été dilué avec de l'éther et de l'eau, la phase organique a été lavée avec une solution aqueuse saturée de NaHCO₃ et de la saumure, puis séchée sur Na₂SO₄ et concentrée pour obtenir 1,60 g d'une huile jaune. Une distillation sur four à boules (0,4x10² Pa, température de four 120°) a permis d'obtenir 0,79 de 2,5,6-triméthyl-2-indaneméthanol avec une pureté de 91%, contenant encore 7% de diol intermédiaire. Une chromatographie « flash » sur SiO₂ (79 g) en utilisant comme éluant un mélange toluène-éthylacétate 4 : 1 a permis d'obtenir 0,69 g de produit purifié. Une distillation sur four à boules (0,7x10² Pa, température de four 120°) a fourni, sous forme d'huile incolore, 0,6 g de produit désiré avec une pureté de 99% et un rendement de 47%.

### Données analytiques :

IR(CHCl₃) : 3590, 3440(large), 2900, 1480, 1440, 1020 cm⁻¹
RMN(¹H, 360 MHz, CDCl₃) : 6,94(s, 2H) ; 3,50(s, 2H) ; 2,84(d, J=16, 2H) 2,58(d, J=16, 2H) ; 2,21(s, 6H) ; 1,67(large, OH) ; 1,16(s, 3H)
RMN(¹³C, 90,5 MHz, CDCl₃) : 140,0(2s) ; 134,4(2s) ; 126,0(2d) ; 70,8(t) ; 45,0(s) ; 42,5(2t) ; 24,1(q) ; 19,7(2q)
SM : 190(M⁺, 45), 172(16), 157(100), 142(17), 128(20), 115(11), 105(6), 91(7), 77(5), 51(3), 39(4), 31(4), 27(2)

### Exemple 4

### Préparation du 2,4,6-triméthyl-2-indaneméthanol

La synthèse du 2,4,6-triméthyl-2-indaneméthanol a été effectuée en deux étapes à partir de la 2,4,6-triméthyl-1-indanone. La synthèse de cette dernière a été décrite par J. Colonge et al. dans Bull. Soc. Chim. Fr., 1951, p. 961.

### a) Préparation de la 2-hydroxyméthyl-2,4,6-triméthyl-1-indanone à partir de la 2,4,6-triméthyl-1-indanone

Un mélange sous agitation de 0,53 g (3,04 mmol) de 2,4,6-triméthyl-1-indanone et 0,21 g (1,52 mmol) de K₂CO₃ dans 5 ml de toluène a été chauffé à 50°; 0,18 ml (3,34 mmol) d'une solution à 55% de formaldéhyde dans du méthanol (Formcel ® 55) lui ont été ajoutés goutte à goutte durant 1 min. Le mélange ainsi obtenu a été maintenu sous agitation à 50° durant 2 h. Une fois refroidi, le mélange a ensuite été dilué avec de l'éther et de la saumure, la phase organique a été lavée 2 fois avec de la saumure, puis séchée sur Na₂SO₄ et concentrée pour obtenir 0,83 g d'une huile jaunâtre. Une cristallisation à partir d'éther à -30° a permis d'obtenir 0,45 g de cristaux cubiques incolores (p. f. 110°) avec un rendement de 73%.

### Données analytiques :

IR(pur) : 3396, 2914, 2869, 1674, 1615, 1482, 1380, 1309, 1250, 1047, 993, 871 cm⁻¹
RMN(¹H, 360 MHz, CDCl₃) : 7,35(s, 1H) ; 7,25(s, 1H) ; 3,82(dd, J₁=10, J₂=7, 1H) ; 3,62(dd, J₁=10, J₂=4, 1H) ; 3,08(d, J=17, 1H) ; 2,73(d, J=17, 1H) ; 2,56(m, OH) ; 2,35(s, 3H) ; 2,31(s, 3H) ; 1,23(s, 3H)
RMN(¹³C, 90,5 MHz, CDCl₃) : 211,6(s) ; 149,8(s) ; 137,6(s) ; 137,0(d) ; 135,7(s) ; 135,5(s) ; 68,0(t) ; 51,1(s) ; 36,6(t) ; 21,0(q) ; 20,8(q) ; 17,7(q)
SM : 204(M⁺, 55), 189(18), 186(58), 173(100), 159(54), 143(28), 128(28), 115(24), 105(8), 91(13), 77(8), 63(4), 51(4), 39(4)

### b) Préparation du 2,4,6-triméthyl-2-indaneméthanol à partir de la 2-hydroxyméthyl-2,4,6-triméthyl-1-indanone

On a ajouté, à une solution de 0,40 g (1,96 mmol) de produit obtenu sous *a)* dans 10 ml d'acide acétique, 0,1 g de Pd-C à 10% et le mélange a été agité vigoureusement sous atmosphère H₂ (10⁵ Pa) à température ambiante durant 36 h. Le catalyseur a été filtré et le filtrat concentré en une huile incolore. Cette huile a été absorbée dans 2 ml de mélange de saponification (2,5 N NaOH dans EtOH-H₂O 1 : 1), puis chauffée à reflux durant 0,5 h. Le mélange refroidi a ensuite été dilué avec de l'éther et NaHCO₃ sat. aq., la phase organique a été lavée 2 fois avec de la saumure, puis séchée sur Na₂SO₄ et concentrée pour obtenir 0,44 d'une huile jaunâtre. Une distillation sur four à boules (0,2x10² Pa, température de four 100-155°) a permis d'obtenir 0,30 g de 2,4,6-triméthyl-2-indaneméthanol sous forme d'une huile incolore, avec une pureté de 98% et un rendement de 78%.

### Données analytiques :

IR(pur) : 3330(large), 2900, 1470, 1370, 1030, 840 cm⁻¹
RMN(¹H, 360 MHz, CDCl₃) : 6,82(s, 1H) ; 6,78(s, 1H) ; 3,50(s, 2H) ; 2,87(d, J=16, 1H) ; 2,78(d, J=16, 1H) ; 2,26(d, J=16, 1H) ; 2,53(d, J=16. 1H) ; 2.28(s, 3H) ; 2,18(s, 3H) ; 1,80(s, OH) ; 1,18(s, 3H)
RMN(¹³C, 90,5 MHz, CDCl₃) : 142,4(s), 138.2(s) ; 136,1(s) ; 133,8(s) ; 128.0(d) ; 122,8(d) ; 70,9(t) ; 44,4(s) ; 42,9(t) ; 41,1(t) ; 24,4(q) ; 21,1(q) ; 19,0(q)
SM : 190(M⁺, 45), 172(14), 157(100), 142(18), 128(20), 115(12), 105(4), 91(5), 77(3), 51(2), 39(2), 31(2), 27(1)

### Exemple 5

### Préparation d'une composition parfumante pour détergent

On a préparé une composition parfumante de base de type fleuri, muguet pour un détergent, en mélangeant les ingrédients suivants :

| Ingrédients | Parties en poids |
|---|---|
| Acétate de citronellyle | 10 |
| Acétate de cis-3-héxénol à 1% * | 20 |
| Aldéhyde hexylcinnamique | 200 |
| Benzylacétone | 20 |
| cis-3-Héxènol à 1% | 20 |
| Citronellol | 60 |
| Formiate de phényléthyle à 10% * | 20 |
| Géraniol | 80 |
| Géranium synth. | 5 |
| Géranyle nitrile ¹⁾ | 5 |
| Indol à 10% * | 10 |
| Linalol | 10 |
| 1-(5,5-diméthyl-1-cyclohexèn-1-yl)-4-pentèn-1-one ²⁾ à 1% * | 20 |
| Méthyl-2-nonynoate à 1%* | 10 |
| Oxyde de rosé à 1% * | 20 |
| Phénethylol ord. | 70 |
| Absolue de rose synth. à 10%* | 25 |
| N-(3-phényl-1-butényl)-antranilate de méthyle ³⁾ * | 10 |
| Vert de Lilas ⁴⁾ | 10 |
| Total | 625 |

| | |
|---|---|
| * dans le dipropylène glycol | |
| 1) 3.7-diméthyl-2,6-octadiènenitrile : origine : Firmenich SA, Genève, Suisse | |
| 2) origine : Firmenich SA, Genève. Suisse | |
| 3) origine : Firmenich SA. Genève, Suisse | |
| 4) (2,2-diméthoxyéthyl)benzène ; origine : Firmenich SA, Genève, Suisse | |

L'addition de 375 parties en poids de 2,5-diméthyl-2-indaneméthanol confère à cette composition de base une superbe connotation muguet fraîche, légèrement verte et poudrée, très rayonnante. L'effet odorant obtenu avec le composé de l'invention est comparable à celui que l'on pourrait obtenir avec le Lilial® [3-(4-tert-butyl-1-phényl)-2-méthylpropanal ; origine : Givaudan-Roure SA] tout en restant beaucoup plus stable que celui de ce dernier dans des détergents, notamment ceux contenant du TAED.

### Exemple 6

### Préparation d'une composition parfumante

On a préparé une composition de base de type fleuri, au caractère lilas à partir des ingrédients suivants :

| Ingrédients | Parties en poids |
|---|---|
| Aldéhyde anisique | 30 |
| Alcool cinnamique ord. à 50%* | 160 |
| Hédione® ¹⁾ HC | 10 |
| Indol à 10%* | 20 |
| Linalol | 200 |
| Phénéthylol | 100 |
| Terpinéol alpha | 350 |
| Vert de Lilas ²⁾ | 30 |
| Total | 900 |

| | |
|---|---|
| * dans le dipropylène glycol | |
| 1) dihydrojasmonate de méthyle à haute teneur en isomère cis ; origine : Firmenich SA, Genève, Suisse | |
| 2) (2,2-diméthoxyéthyl)benzène ; origine : Firmenich SA, Genève, Suisse | |

L'addition de 100 parties en poids de 2,5-diméthyl-2-indaneméthanol à cette composition de base a permis d'obtenir une composition nouvelle dont l'odeur possédait une jolie connotation florale-lilas, beaucoup plus puissante, plus naturelle et rayonnante que celle de la composition de base.

### Exemple 7

### Préparation d'une composition parfumante pour une eau de toilette pour femme

On a préparé une composition de base destinée à une eau de toilette pour femme, à partir des ingrédients suivants :

| Ingrédients | Parties en poids |
|---|---|
| Acétate de benzyle | 80 |
| Acétate de styrallyle | 5 |
| Ambrettolide | 30 |
| Astrotone ¹⁾ | 550 |
| Cassis base 345 B ²⁾ | 35 |
| Cétalox® ³⁾ | 15 |
| cis-Jasmone | 15 |
| (Cyclohexyloxy)-acétate d'allyle ⁴⁾ à 10%* | 180 |
| Ethyl linalol | 250 |
| Absolue de feuilles de violette à 10% * | 10 |
| 2-Méthyl-4-(2,2,3-triméthyl-3-cyclopenten-1-yl)- | |
| 4-pentèn-1-ol ⁵⁾ à 10% * | 25 |
| Galaxolide® 50 MIP ⁶⁾ | 1500 |
| Habanolide® ⁷⁾ | 210 |
| Hédione® HC ⁸⁾ | 2000 |
| Hélional ⁹⁾ | 200 |
| Indolène | 50 |
| Iralia® ¹⁰⁾ | 130 |
| Iso E Super ¹¹⁾ | 200 |
| Levocitrol | 24 |
| 3-Méthyl-cyclopentadécénone ¹²⁾ | 100 |
| Essence de patchouli | 5 |
| Phénethylol | 190 |
| Essence de poivre | 50 |
| Triplal® ¹³⁾ | 15 |
| Total | 6000 |

| | |
|---|---|
| *dans le dipropylène glycol | |
| 1) 1,4-dioxa-5,17-cycloheptadécanedione ; origine : Firmenich SA, Genève, Suisse | |
| 2) origine : Firmenich SA, Genève, Suisse | |
| 3) 8,12-époxy-13,14,15,16-tétranorlabdane ; origine : Firmenich SA, Genève, Suisse | |
| 4) origine : Firmenich SA, Genève, Suisse | |
| 5) origine : Firmenich SA, Genève, Suisse | |
| 6) 1,3,4,6,7,8-héxahydro-4,6,6,7,8,8-hexaméthyl-cyclopenta-g-2-benzopyrane ; origine : International Flavors & Fragrances Inc., USA | |
| 7) pentadécénolide ; origine : Firmenich SA, Genève, Suisse | |
| 8) dihydrojasmonate de méthyle à haute teneur en isomère cis ; origine : Firmenich SA, Genève, Suisse | |
| 9) 3-(1,3-benzodioxol-5-yl)-2-méthylpropanal: origine : Firmenich SA, Genève, Suisse | |
| 10) méthylionone ; origine : Firmenich SA, Genève, Suisse | |
| 11) 1-(octahydro-2,3,8,8-tétraméthyl-2-naphtalényl)-1-éthanone ; origine : International Flavors & Fragrances Inc., USA | |
| 12) origine : Firmenich SA, Genève, Suisse | |
| 13) 2,4-diméthyl-3-cyclohex-1-èn-1-carboxaldéhyde; origine : International Flavors & Fragrances Inc., USA | |

Lorsqu'on a ajouté 700 parties en poids de 2,5-diméthyl-2-indaneméthanol à cette eau de toilette moderne, on a obtenu une composition nouvelle à odeur florale, transparente, dont la note de fond était encore plus rayonnante.

### Exemple 8

### Préparation d'une composition parfumante pour une eau de toilette pour femme

On a préparé une composition de base destinée à une eau de toilette pour femme, à partir des ingrédients suivants :

| Ingrédients | Parties en poids |
|---|---|
| Acétate de vétyvéryle | 1000 |
| Aldéhyde hexylcinnamique | 120 |
| Amione®¹⁾ | 5 |
| Benzophénone | 15 |
| Essence de bergamote | 120 |
| Cedroxyde® ²⁾ | 50 |
| Cetalox® ³⁾ à 10%* | 40 |
| Essence de citron de Californie | 20 |
| Citronellol | 110 |
| Coumarine | 20 |
| Essence de galbanum à 10% * | 120 |
| Géraniol | 150 |
| Essence de géranium de Chine naturel | 40 |
| Essence de girofle anglaise | 20 |
| Hédione®⁴⁾ HC | 580 |
| Indol purifié à 1% * | 40 |
| Iralia®⁵⁾ | 460 |
| Absolue d'iris synth. | 20 |
| Isobutylquinoléine ⁶⁾ à 10% * | 40 |
| Absolue de mousse de chêne Dalma à 50% * | 20 |
| Néroli bigarade pétales | 20 |
| Absolue d'opoponax | 20 |
| Phénethylol ord. | 580 |
| Absolue de rose | 30 |
| Essence de santal | 100 |
| Undécalactone à 10% * | 20 |
| Vanilline à 10% * | 40 |
| Vertofix coeur⁷⁾ | 240 |
| Ylang | 160 |
| Total | 4300 |

| | |
|---|---|
| * dans le dipropylène glycol | |
| 1) allyl ionone ; origine : Givaudan-Roure SA, Vernier, Suisse | |
| 2) triméthyl-13-oxabicyclo-[10.1.0]-tridéca-4,8-diène ; Firmenich SA, Genève, Suisse | |
| 3) 8,12-époxy-13,14,15,16-tétranorlabdane ; origine : Firmenich SA, Genève. Suisse | |
| 4) dihydrojasmonate de méthyle ; origine : Firmenich SA, Genève, Suisse | |
| 5) mélange d'iso-méthyl ionones ; origine : Firmenich SA, Genève, Suisse | |
| 6) origine : International Flavors & Fragrances Inc., USA | |
| 7) origine : International Flavors & Fragrances Inc., USA | |

L'adjonction de 400 parties en poids de 2,5-diméthyl-2-indaneméthanol à cette composition de base lui confère une note florale fraîche et augmente nettement l'impact de la note de tête du parfum, tout en accroissant sa diffusion au cours de l'évaporation.

## Revendications

1. Composé de formule 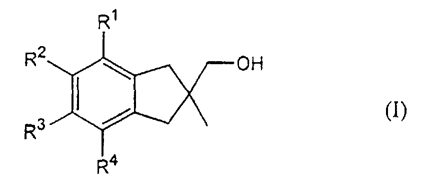 dans laquelle les symboles R¹, R², R³ et R⁴ représentent, indépendamment l'un de l'autre, un atome d'hydrogène ou un radical alkyle inférieur de C₁ à C₃, linéaire ou ramifié, saturé ou insaturé, pour autant que la combinaison selon laquelle R¹=R²=R³=R⁴=H soit exclue.

2. Composé selon la revendication 1, **caractérisé en ce que** seuls deux symboles choisis parmi R¹, R², R³ et R⁴ représentent un atome d'hydrogène.

3. Composé selon la revendication 1, **caractérisé en ce que** seul un des symboles choisis parmi R¹, R², R³ et R⁴ représente un atome d'hydrogène.

4. Composé selon l'une des revendications 2 ou 3, **caractérisé en ce que** les symboles ne représentant pas un atome d'hydrogène, représentent un radical méthyle.

5. A titre de composé selon la revendication 1, un composé choisi dans le groupe constitué par le 2,5-diméthyl-2-indaneméthanol, le 2,4-diméthyl-2-indaneméthanol, le 2,5,6-triméthyl-2-indaneméthanol et le 2,4,6-triméthyl-2-indaneméthanol.

6. A titre de composé selon la revendication 5, le 2,5-diméthyl-2-indane-méthanol, sous forme racémique ou sous forme d'un isomère optiquement actif.

7. Utilisation d'un composé selon l'une des revendications 1 à 6 à titre d'ingrédient parfumant.

8. Composition parfumante ou article parfumé contenant en tant qu'ingrédient actif un composé selon l'une des revendications 1 à 6.

9. Article parfumé selon la revendication 8 sous la forme d'un parfum ou d'une eau de toilette, d'une lotion après rasage, d'une préparation cosmétique, d'un savon, d'un shampoing ou après-shampoing ou autre produit de soin capillaire, d'un gel de bain ou de douche, d'un déodorant corporel ou d'un déodorant ambiant, d'un détergent ou adoucissant textile ou d'un produit d'entretien.

10. Procédé pour la préparation d'un composé selon la revendication 1, **caractérisé en ce qu'**on soumet à une hydrogénolyse une indanone de formule 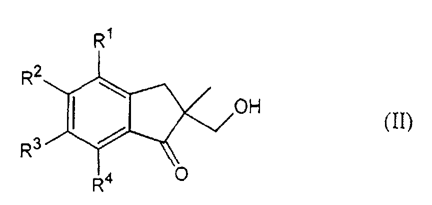 dans laquelle les symboles R¹, R², R³ et R⁴ sont définis comme à la revendication 1.

11. Composé de formule (II) telle que définie à la revendication 10.

12. Composé selon la revendication 11, **caractérisé en ce que** les symboles R¹ à R⁴ dans la formule (II) sont définis comme à la revendication 2, 3 ou 4.

13. Composé selon la revendication 11, choisi dans le groupe constitué par la 2-hydroxyméthyl-2,5-diméthyl-1-indanone, 2-hydroxyméthyl-2,4-diméthyl-1-indanone, 2-hydroxyméthyl-2,5,6-triméthyl-1-indanone et 2-hydroxyméthyl-2,4,6-triméthyl-1-indanone.

## Claims

1. A compound of the formula 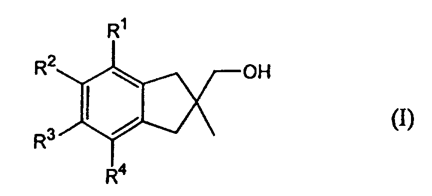 wherein the symbols R¹, R², R³ and R⁴ represent, independently of one another, a hydrogen atom or a linear or branched, saturated or unsaturated C₁ to C₃ lower alkyl radical, provided that the combination in which R¹=R²=R³=R⁴=H is excluded.

2. A compound according to claim 1, **characterised in that** only two symbols selected from R¹, R², R³ and R⁴ represent a hydrogen atom.

3. A compound according to claim 1, **characterised in that** only one of the symbols selected from R¹, R², R³ and R⁴ represents a hydrogen atom.

4. A compound according to either one of claims 2 or 3, **characterised in that** the symbols not representing a hydrogen atom represent a methyl radical.

5. As a compound according to claim 1, a compound selected from the group consisting of 2,5-dimethyl-2-indanmethanol, 2,4-dimethyl-2-indanmethanol, 2,5,6-trimethyl-2-indanmethanol and 2,4,6-trimethyl-2-indanmethanol.

6. As a compound according to claim 5, 2,5-dimethyl-2-indanmethanol in racemic form or in the form of an optically active isomer.

7. Use of a compound according to any one of claims 1 to 6 as a perfuming ingredient.

8. A perfuming composition or perfumed article containing a compound according to any one of claims 1 to 6 as an active ingredient.

9. A perfumed article according to claim 8, in the form of a perfume or eau de toilette, after-shave lotion, cosmetic preparation, soap, shampoo or conditioner or other hair care product, bath gel or shower gel, deodorant or air freshener, detergent or fabric softener or household product.

10. A process for the preparation of a compound according to claim 1, **characterised by** the hydrogenolysis of an indanone of formula 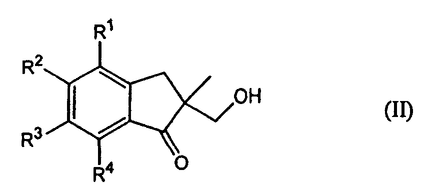 wherein the symbols R¹, R², R³ and R⁴ are defined as in claim 1.

11. A compound of formula (II) as defined in claim 10.

12. A compound according to claim 11, **characterised in that** the symbols R¹ to R⁴ in formula (II) are defined as in claim 2, 3 or 4.

13. A compound according to claim 11, selected from the group consisting of 2-hydroxymethyl-2,5-dimethyl-1-indanone, 2-hydroxymethyl-2,4-dimethyl-1-indanone, 2-hydroxymethyl-2,5,6-trimethyl-1-indanone and 2-hydroxymethyl-2,4,6-trimethyl-1-indanone.

## Patentansprüche

1. Verbindung der Formel 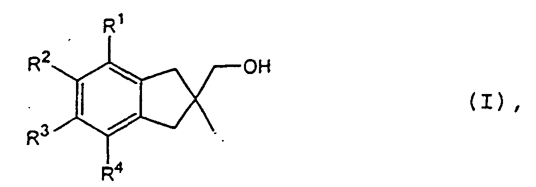 in der die Symbole R¹, R², R³ und R⁴ unabhängig voneinander für ein Wasserstoffatom oder einen geradkettigen oder verzweigten, gesättigten oder ungesättigten niederen C₁-C₃-Alkylrest stehen, unter der Maßgabe, daß die Kombination, nach welcher R¹=R²=R³=R⁴=H, ausgeschlossen ist.

2. Verbindung nach Anspruch 1, **dadurch gekennzeichnet, daß** nur zwei unter R¹, R², R³ und R⁴ ausgewählte Symbole für ein Wasserstoffatom stehen.

3. Verbindung nach Anspruch 1, **dadurch gekennzeichnet, daß** nur eines der unter R¹, R², R³ und R⁴ ausgewählten Symbole für ein Wasserstoffatom steht.

4. Verbindung nach einem der Ansprüche 2 oder 3, **dadurch gekennzeichnet, daß** die Symbole, die nicht für ein Wasserstoffatom stehen, für einen Methylrest stehen.

5. Als Verbindung gemäß Anspruch 1, eine Verbindung, die aus der Gruppe bestehend aus 2,5-Dimethyl-2-indanmethanol, 2,4-Dimethyl-2-indanmethanol, 2,5,6-Trimethyl-2-indanmethanol und 2,4,6-Trimethyl-2-indanmethanol ausgewählt ist.

6. Als Verbindung gemäß Anspruch 5, 2,5-Dimethyl-2-indanmethanol in racemischer Form oder in Form eines optisch aktiven Isomers.

7. Verwendung einer Verbindung nach einem der Ansprüche 1 bis 6 als Riechstoff.

8. Duftzusammensetzung oder parfümierter Artikel, welche bzw. welcher als Riechstoff eine Verbindung nach einem der Ansprüche 1 bis 6 enthält.

9. Parfümierter Artikel nach Anspruch 8 in Form eines Parfüms oder eines Eau de Toilette, einer After-Shave-Lotion, eines kosmetischen Präparats, einer Seife, eines Shampoo oder nach der Haarwäsche anzuwendenden oder anderen Haarpflegeproduktes, eines Bade- oder Duschgels, eines Körper- oder Raumluftdeodorants, eines betergens oder Textilweichspülers oder eines Allzweckreinigers.

10. Verfahren zur Herstellung einer Verbindung gemäß Anspruch 1, **dadurch gekennzeichnet, daß** ein Indanon der Formel 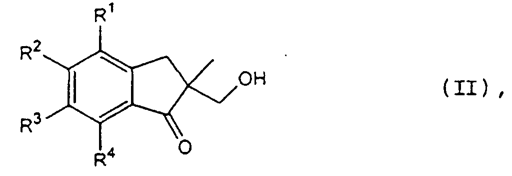 in der die Symbole R¹, R², R³ und R⁴ wie in Anspruch 1 definiert sind, einer Hydrogenolyse unterzogen wird.

11. Verbindung der Formel (II) gemäß der Definition in Anspruch 10.

12. Verbindung nach Anspruch 11, **dadurch gekennzeichnet, daß** die Symbole R¹ bis R⁴ in der Formel (II) wie in Anspruch 2, 3 oder 4 definiert sind.

13. Verbindung nach Anspruch 11, ausgewählt aus der Gruppe bestehend aus 2-Hydroxymethyl-2,5-dimethyl-1-indanon, 2-Hydroxymethyl-2,4-dimethyl-1-indanon, 2-Hydroxymethyl-2,5,6-trimethyl-1-indanon und 2-Hydroxymethyl-2,4,6-trimethyl-1-indanon.
